# EUROPÄISCHE PATENTSCHRIFT

(11) **EP 1 934 423 B1**
(45) Veröffentlichungstag und Bekanntmachung des Hinweises auf die Patenterteilung: **18.12.2013**
(21) Anmeldenummer: 06804343.9
(22) Anmeldetag: 10.10.2006
(51) Int. Cl.: E05D 15/40

(54) **KLAPPENBESCHLAG**
SHUTTER (OR DOOR) FITTING
FERRURE POUR ABATTANT

(30) Priorität: 12.10.2005 AT 16592005
(43) Veröffentlichungstag der Anmeldung: 25.06.2008
(73) Patentinhaber: Julius Blum GmbH, 6973 Höchst (AT)
(72) Erfinder: DUBACH, Fredi, CH-8344 Bäretwil (CH); BRUNNMAYR, Harald, A-6912 Hörbranz (AT)
(74) Vertreter: Hofinger, Stephan
(86) Internationale Anmeldenummer: PCT/AT2006/000415
(87) Internationale Veröffentlichungsnummer: WO 2007/041736

(56) Entgegenhaltungen:
- EP-A- 1 296 011
- DE-A1- 2 752 186
- FR-A- 2 089 842

## Beschreibung

Die vorliegende Erfindung bezieht sich auf einen Klappenbeschlag zum schwenkbaren Befestigen einer Möbelklappe an einem Schrankkorpus, mit mindestens einem Stellarm, der zum Bewegen der Möbelklappe vorgesehen ist, und mit mindestens einem klappenseitigen Beschlagteil, der mit dem Stellarm verbindbar ist, wobei der klappenseitige Beschlagteil eine Verdrehsicherung zum zeitweiligen Fixieren der Schwenklage des mindestens einen Stellarmes, vorzugsweise in dessen vollständiger Offenstellung, aufweist, wobei die Verdrehsicherung in einer ersten Betriebsstellung den mindestens einen Stellarm in seiner Schwenklage relativ zum klappenseitigen Beschlagteil arretiert und in einer zweiten Betriebsstellung eine Schwenkbewegung des Stellarmes erlaubt. Ein solcher Beschlag ist Z.B. aus der DE-A-27 52186 bekannt.

Derartige Klappenbeschläge fiinden beispielsweise bei Oberschränken Verwendung, sodass sich die Möbelklappe in Bezug zum Möbelkorpus nach oben hin öffnen lässt. Der Stellarm wird dabei mit einem Endbereich am Möbelkorpus oder an einem korpusseitigen Beschlagteil befestigt, während der andere Endbereich des Stellarmes am klappenseitigen Beschlagteil angelenkt ist. Die Möbelklappe wird einerseits mittels Scharnieren am Möbelkorpus befestigt und andererseits mit dem Stellarm verbunden, der zum Bewegen der Möbelklappe von einer Schließ- in eine Offenstellung vorgesehen ist. Die Montage der Möbelklappe in Bezug zum Stellarm wird durch den sich schwenkenden Stellarm erschwert, da dieser instabil ist und beim Anbringen der Klappe in das Innere des Möbelkorpus abgleiten kann.

Aus der DE 27 52 186 ist ein Parallelschwingbeschlag mit einer daran angelenkten Ablage bekannt geworden, die zwischen einer Schließstellung und einer Offenstellung in einer ständig horizontal geführten Lage verschwenkbar ist. Um ein unbeabsichtiges Verschwenken der Ablage aus den Endlagen zu vermeiden, ist ein schwenkbarer Rasthebel vorgesehen, der mit an den Stellarmen angeordneten Rastbolzen verrastbar ist.

Aufgabe der vorliegenden Erfindung ist es daher, einen Klappenbeschlag der eingangs erwähnten Gattung vorzuschlagen, der eine komfortable Montage der Möbelklappe erlaubt.

Dies wird erfindungsgemäß in einer vorteilhaften Ausgestaltung dadurch erreicht, dass die Verdrehsicherung ein Verrisgelteil aufweist, der in der arretierenden ersten Betriebsstellung in einer Ausnehmung eingreift, welche an einem um eine Drehachse des Stellarmes drehbar gelagerten Lagerbolzen oder an einem um eine Drehachse des Stellarmes drehbar gelagerten Umlenkteil angeordnet ist.

Gemäß eines ersten Aspekts der Erfindung geht es darum, die Montage der Möbelklappe relativ zum klappenseitigen Beschlagteil zu erleichtern. Zu diesem Zweck wird die relative Lage des klappenseitigen Beschlagteiles in Bezug zum Stellarm durch die vorgeschlagene Verdrehsicherung arretiert.

Gemäß einem weiteren Aspekt der Erfindung geht es aber auch darum, nicht nur die relative Lage des klappenseitigen Beschlagteiles zum Stellarm festzulegen, sondern gleichzeitig auch die relative Lage des Stellarmes in Bezug zum Schrankkorpus zu arretieren. Dabei wird der klappenlose bzw. "leere" Stellarm vorzugsweise in seiner vollständigen Offenstellung hinsichtlich seiner Schwenklage fixiert, sodass dieser bei der Montage der Möbeiklappe nicht abgleiten bzw. durch die übliche Federvorrichtung (die den Stellarm zum Gewichtsausgleich der Klappe zum Teil mit sehr hohen Kräften beaufschlagt) wieder in die Höhe schnellen und dabei Verletzungen des Montagepersonals verursachen kann.

Gemäß einer ersten Variante der Erfindung kann vorgesehen sein, dass auf jeder Seitenwand des Schrankkorpus wenigstens zwei Stellarme mit jeweils zwei Drehachsen zum Bewegen der Möbelklappe vorgesehen sind, wobei durch die Verdrehsicherung wenigstens eine Drehachse der Stellarme arretierbar ist. Dabei kann es zweckmäßig sein, wenn die wenigstens zwei Stellarme mit dem klappenseitigen Beschlagteil ein Viergelenk bilden. Wird bei einem derartigen Viergelenk eine Drehachse arretiert, so werden dabei auch die anderen Drehachsen in ihrer Lage fixiert, sodass das gesamte Hebelwerk zum Bewegen der Möbelklappe für den Montageprozess unbeweglich ist.

Gemäß einer alternativen Variante der Erfindung kann auch vorgesehen sein, dass auf jeder Seitenwand des Schrankkorpus lediglich ein Stellarm mit zwei Drehachsen zum Bewegen der Möbelklappe vorgesehen ist, wobei mindestens ein zusätzliches Seil, vorzugsweise ein Zahnriemen, zwischen einem drehfest am Schrankkorpus gelagerten Umlenkteil und einem weiteren Umlenkteil geführt ist, der drehbar am freien Ende des Stellarmes gelagert ist und der drehfest mit dem klappenseitigen Beschlagteil verbindbar ist, wobei durch die Verdrehsicherung der weitere Umlenkteil relativ zum Stellarm arretierbar ist. Mit anderen Worten wird - wenn nur ein Stellarm zum Bewegen der Klappe vorgesehen ist - durch ein zusätzliches Seil bzw. einen Zahnriemen eine Zwangsführung zwischen dem drehfest am Schrankkorpus angeordneten Umlenkteil und dem drehbar am freien Ende des Stellarmes gelagerten Umlenkteil herbeigeführt. Für die Klappenmontage ist es günstig, wenn die Verdrehsicherung einen am Stellarm angeordneten Schieber aufweist, der mit dem weiteren Umlenkteil des klappenseitigen Beschlagteiles in Eingriff bringbar ist.

Nach erfolgter Montage der Klappe wird die Verdrehsicherung deaktiviert, sodass der übliche Bewegungsablauf der Klappe nicht behindert wird.

Gemäß einem Ausführungsbeispiel der Erfindung kann vorgesehen sein, dass der Stellarm eine Drehachse mit einer Ausnehmung aufweist, in die ein Verriegelteil einbringbar ist. Dabei kann die Ausgestaltung derart getroffen sein, dass die Ausnehmung radial zur Drehachse des Stellarmes verläuft. Alternativ kann es auch zweckmäßig sein, wenn die Ausnehmung quer zur Drehachse des Stellarmes verläuft.

Bei einer vorteilhaften Ausführungsform der Erfindung kann vorgesehen sein, dass der Verriegelteil von einem Schieber gebildet ist. Es liegt jedoch auch im Rahmen der Erfindung, dass der Verriegelteil von einem drehbaren Teil gebildet ist.

Gemäß einem bevorzugten Ausführungsbeispiel der Erfindung kann vorgesehen sein, dass der klappenseitige Beschlagteil einen an der Klappe vormontierten Grundkörper und einen mit dem Stellarm verbundenen Befestigungsteil aufweist. Dabei kann die Ausgestaltung so getroffen sein, dass der Befestigungsteil mit dem Grundkörper durch eine lösbare Befestigungsvorrichtung, vorzugsweise eine mechanische Rastverbindung, verbindbar ist. In diesem Zusammenhang kann es auch zweckmäßig sein, wenn die mechanische Rastverbindung derart ausgebildet ist, dass der Befestigungsteil in den Grundkörper einhängbar und anschließend durch Aufschwenken mit diesem verrastbar ist.

Auf diese Weise wird eine komfortable Befestigung der Klappe in Bezug zum Stellarm geschaffen, da der Stellarm in seiner Offenstellung fixiert ist und die Möbelklappe mit ihrem vormontierten Grundkörper in einfacher Weise am Befestigungsteil des Stellarmes anküppbar ist.

Gemäß einem bevorzugten Ausführungsbeispiel der Erfindung kann vorgesehen sein, dass der Befestigungsteil mit dem Grundkörper zusätzlich zur lösbaren Befestigungsvorrichtung durch eine gesonderte Abhebesicherung gesichert ist. Bei Verwendung einer mechanischen Rastverbindung, insbesondere mit einem federbelasteten Cliphebel, besteht die Möglichkeit, dass dieser unbeabsichtigt betätigt wird, was zu einem Lösen bzw. Absturz der Klappe vom Klappenbeschlag führen würde. Durch das Vorsehen einer zusätzlichen Abhebesicherung wird die Klappe gegen Absturz doppelt gesichert und ist somit unverlierbar am Klappenbeschlag gehaltert.

Gemäß einem bevorzugten Ausführungsbeispiel der Erfindung kann vorgesehen sein, dass die Abhebesicherung einen vorzugsweise federbeaufschlagten, Schieber umfasst, der in einer Betriebsstellung mit einem am Grundkörper oder am Befestigungsteil angeordneten oder ausgebildeten Sicherungsbügel in Eingriff bringbar ist. In diesem Zusammenhang kann eine konstruktiv einfache Ausgestaltung so getroffen sein, dass die Verdrehsicherung vom selben Schieber wie die Abhebesicherung gebildet ist.

Ein weiteres bevorzugtes Ausführungsbeispiel sieht vor, dass die relative Lage des Stellarmes in Bezug zum klappenseitigen Beschlagteil durch wenigstens eine Einstellvorrichtung veränderbar ist. Bedingt durch Fertigungstoleranzen und geringfügigen Einbaufehlern kann es zur Ausbildung eines unattraktiven äußeren Fugenbildes kommen, da die Möbelklappe in Bezug zum Möbelkorpus bzw. zu Möbelklappen benachbarter Schränke nicht optimal ausgerichtet ist. Auf diese Weise wird im Gegensatz zu einer gemäß dem Stand der Technik bekannten Ausführungsform, bei der der Stellarm lediglich drehgelenkig mit der Klappe verbunden ist, eine aktive Beeinflussung der relativen Lage zwischen Stellarm (bzw. einem Lagerteil desselben) und dem klappenseitigen Beschlagteil ermöglicht. Die Einstellvorrichtung ermöglicht dem Montagepersonal eine individuelle und einfach durchzuführende Justierung des Klappenbeschlages, um damit ein optisch ansprechendes Fugenbild herbeizuführen.

Gemäß einem bevorzugten Ausführungsbeispiel der Erfindung kann vorgesehen sein, dass durch die wenigstens eine Einstellvorrichtung der Stellarm oder dessen Lagerteil in Bezug zum klappenseitigen Beschlagteil in seiner Höhe und/oder in seiner Neigung und/oder in seiner seitlichen Ausrichtung verstellbar ist. Damit wird eine unabhängige dreidimensionale Verstellmöglichkeit des Stellarms bzw. dessen Lagerteiles in Bezug zum klappenseitigen Beschlagteil bzw. zur Klappe ermöglicht, die eine optimale Justierung der bereits montierten Klappe erlaubt. In diesem Zusammenhang kann die Ausgestaltung so getroffen sein, dass für die Verstellung der Höhe, der Neigung und der seitlichen Ausrichtung jeweils eine gesonderte Einstellvorrichtung vorgesehen ist.

Durch die gesonderten Einstellvorrichtungen wird eine dreidimensionale Einstellung der Lagerpunktlage des wenigstens einen Stellarmes relativ zum klappenseitigen Grundkörper (welcher vorzugsweise fest mit der Klappe verschraubt ist) möglich. Die Verstellung der Höhe H bewirkt eine Änderung der vorstehend genannten Lagerpunktlage in Längserstreckung der Möbelklappe, während die Verstellung der seitlichen Ausrichtung B eine Änderung der Lagerpunktlage entlang der Breite der Möbelklappe bewirkt. Die Verstellung der Höhe H und der seitlichen Ausrichtung B erfolgt also vorzugsweise in zweidimensionaler XY-Richtung parallel zur Klappenebene, während die zusätzliche Verstellung der Neigung α eine Neigungsverstellung des Stellarmes bzw. dessen Lagerteiles relativ zum klappenseitigen Beschlagteil bewirkt, was in weiterer Folge zu einer Neigungsänderung der Klappenebene relativ zur Stirnseitenebene des Schrankkorpus führt.

Bei einem weiteren vorteilhaften Ausführungsbeispiel der Erfindung kann vorgesehen sein, dass am Befestigungsteil mittelbar oder unmittelbar zwei Stellarme schwenkbar gelagert sind. Hierbei können vorzugsweise ein Stellarm und ein Steuerarm Verwendung finden, wobei der Stellarm korpusseitig üblicherweise von einer Federvorrichtung zum Gewichtsausgleich der Klappe beaufschlagt ist. Der Steuerarm hingegen bestimmt dabei den Bewegungsablauf, d.h. die Lage der Klappe in Bezug zum Möbelkorpus während ihrer Öffnungs- und Schließbewegung.

Eine konstruktiv einfache Ausgestaltung der Erfindung sieht vor, dass die wenigstens eine Einstellvorrichtung mindestens eine Gewindeschraube aufweist, die manuell oder durch ein Verstellelement, vorzugsweise einen Schraubendreher, betätigbar ist. In diesem Zusammenhang ist es selbstverständlich auch möglich, anstelle der Gewindeschraube Exzenter bzw. Scheiben vorzusehen, die exzentrisch auf einer Welle befestigt sind. Auch kann die Ausgestaltung so getroffen sein, dass die Einstellvorrichtung(en) wenigstens ein, vorzugsweise selbsthemmendes, Schneckengetriebe aufweist (aufweisen).

Weitere Einzelheiten und Vorteile der vorliegenden Erfindung werden anhand der Figurenbeschreibung unter Bezugnahme auf die Zeichnungen im Folgenden näher erläutert. Darin zeigt bzw. zeigen:
- Fig. 1a, 1b: eine Seitenansicht eines schrankförmigen Möbels mit einer nach oben zu öffnenden Klappe sowie eine Detaildarstellung des Klappenbeschlages,
- Fig. 2a-2c: den klappenseitigen Klappenbeschlag in einer Explosionsdarstellung, im zusammengebauten Zustand sowie in einer perspektivischen Darstellung von hinten,
- Fig. 3a, 3b: eine perspektivische Darstellung des Klappenbeschlages mit verdrehgesicherter Drehachse sowie einen Vertikalschnitt desselben,
- Fig. 4a, 4b: eine perspektivische Darstellung des Klappenbeschlages mit aktiver Abhebesicherung sowie einen Vertikalschnitt desselben,
- Fig. 5a-5c: verschiedene Ansichten des vollständig verrasteten Klappenbeschlages,
- Fig. 6a-6d: den Klappenbeschlag in verschiedenen Ansichten bei Durchführung einer Höhenverstellung,
- Fig. 7a-7d: den Klappenbeschlag in verschiedenen Ansichten bei Durchführung einer Neigungsverstellung,
- Fig. 8a-8c: den Klappenbeschlag in verschiedenen Ansichten bei Durchführung einer Seitenverstellung,
- Fig. 9a,9b: eine alternative Ausführungsform der Verdrehsicherung mit einem drehbaren Teil zum Arretieren der Drehachse des Stellarmes,
- Fig. 10a, 10b: die Verdrehsicherung aus Fig. 9a, 9b, wobei die Drehachse des Stellarmes arretiert ist,
- Fig. 11a, 11b: die Verdrehsicherung aus Fig. 9a, 9b, wobei die Drehachse des Stellarmes frei bewegbar ist,
- Fig. 12a, 12b: den klappenseitigen Beschlag mit dem drehbaren Teil der Verdrehsicherung, der in einer Stellung den Zutritt eines Schraubendrehers gestattet und in einer zweiten Stellung den Zutritt des Schraubendrehers verhindert,
- Fig. 13a, 13b: Seitenansichten eines am Möbelkorpus montierten Stellmechanismus mit verschwenkgesichertem bzw. verschwenkbarem Stellarm, und
- Fig. 14a, 14b: eine weitere Variante der Erfindung mit nur einem Stellarm zum Bewegen der Klappe, mit der Verdrehsicherung in der passiven bzw. aktiven Stellung.

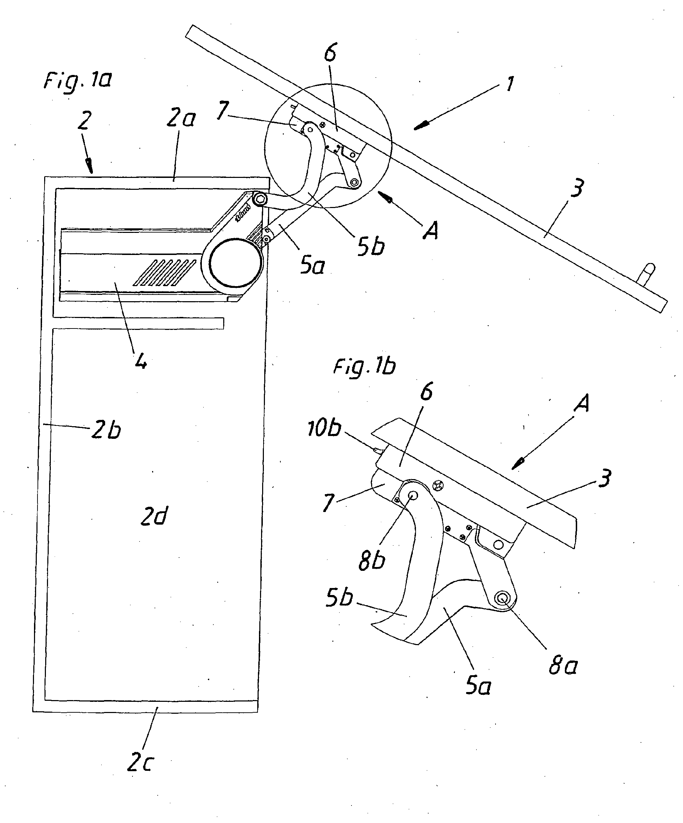

Fig. 1a zeigt eine Seitenansicht eines schrankförmigen Möbels 1 mit einer nach oben hin zu öffnenden Klappe 3. Fig. 1 b zeigt die Detailansicht A aus Fig. 1a. Das schrankförmige Möbel 1 umfasst in herkömmlicher Weise einen Schrankkorpus 2, einen Schrankdeckel 2a, eine Rückwand 2b, einen Schrankboden 2c sowie zwei Seitenwände 2d, auf denen vorzugsweise jeweils ein Stellmechanismus 4 zum Bewegen der Klappe 3 montiert ist. Der Stellmechanismus 4 umfasst eine Feder- bzw. Antriebsvorrichtung, auf die nicht näher Bezug genommen wird. Der Stellmechanismus 4 weist zwei Stellarme 5a und 5b auf, die zum Bewegen der Klappe 3 zwischen einer Schließstellung und einer Offenstellung vorgesehen sind. Die Stellarme 5a, 5b weisen Drehachsen 8a-8d auf, wobei durch eine noch näher zu beschreibende Verdrehsicherung wenigstens eine Drehachse 8a-8d arretierbar ist. Die Stellarme 5a, 5b bilden mit den Drehachsen 8a-8d ein Viergelenk.

Fig. 1b zeigt die Detailansicht A aus Fig. 1a. Auf der Rückseite der Klappe 3 ist ein klappenseitiges Beschlagteil 6 vorgesehen, das über eine lösbare Befestigungsvorrichtung 10b mit dem Lagerteil 7 der beiden Stellarme 5a und 5b mittelbar oder unmittelbar in Verbindung steht. Die beiden Stellarme 5a und 5b sind über Drehachsen 8a, 8b am Lagerteil 7 gelagert, wobei die noch näher zu beschreibende erfindungsgemäße Verdrehsicherung vorzugsweise auf die Drehachse 8b wirkt.

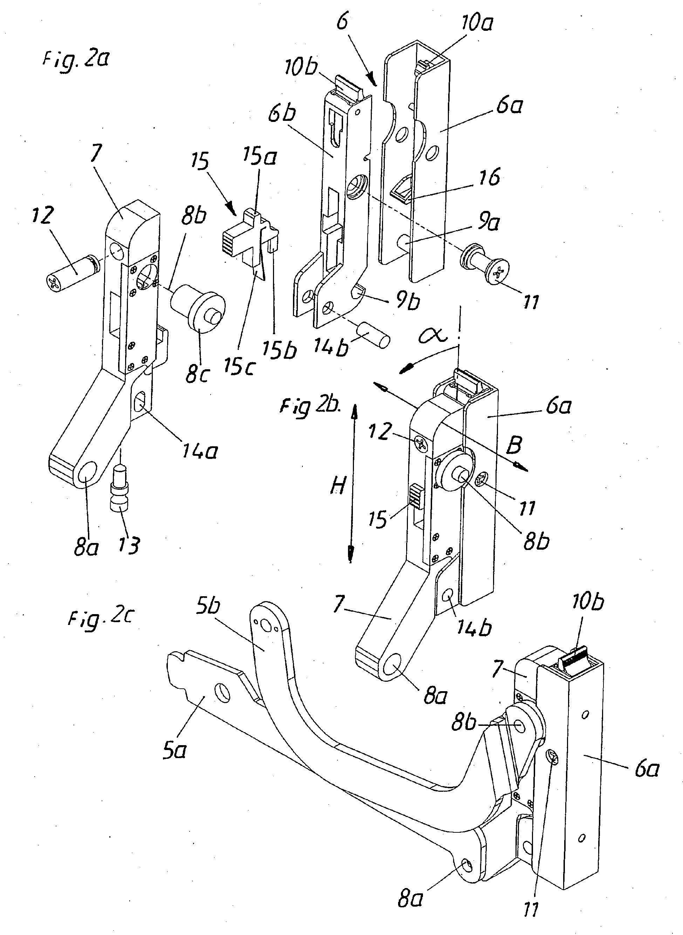

Fig. 2a zeigt eine Explosionsdarstellung eines beispielhaften Klappenbeschlages. Das klappenseitige Beschlagteil 6 ist zweiteilig ausgebildet und umfasst einen an der Klappe 3 vormontierten Grundkörper 6a sowie einen mit den Stellarmen 5a und 5b verbundenen Befestigungsteil 6b, die über eine vorzugsweise federbelastete mechanische Rastverbindung 10a, 10b miteinander verrastbar sind. Der Grundkörper 6a wird an der Klappeninnenseite vormontiert, der Befestigungsteil 6b wird bei der Montage zunächst mit seiner Ausnehmung 9b in den Bolzen 9a eingehängt und durch Aufschwenken mithilfe des Rasthebels 10b in der Rastausnehmung 10a verrastet. Der Befestigungsteil 6b ist etwas schmäler als der U-förmige Grundkörper 6a ausgebildet, sodass der Befestigungsteil 6b mithilfe der Seitenverstellschraube 11 zwischen den Schenkeln des U-förmigen Grundkörpers 6a hin- und herbewegbar ist. Der Bolzen 14b lagert ortsfest am Befestigungsteil 6b, wobei eine Betätigung der Höhenverstellschraube 13 eine Höhenänderung des Lagerteiles 7 in Bezug zum Befestigungsteil 6b herbeiführt. Auf der Drehachse 8b befindet sich der Lagerbolzen 8c, der für die Lagerung des in Fig. 2c gezeigten Stellarmes 5b vorgesehen ist. Die Neigungsverstellschraube 12 durchsetzt den Lagerteil 7 und stützt sich im montierten Zustand am Montageteil 6b ab, um eine Inklination des Lagerteiles 7 in Bezug zum Befestigungsteil 6b herbeizuführen. Der Schieber 15, dessen Funktion in den nachfolgenden Figuren noch näher erläutert wird, umfasst den Verriegelteil 15a (der Teil der Verdrehsicherung für den Stellarm 5b ist) und die Abhebesicherung 15b, die mit dem Sicherungsbügel 16 des Grundkörpers 6a in Eingriff bringbar ist. Der Schieber 15 ist federbeaufschlagt, und zwar durch die Blattfeder 15c, wodurch ein unbeabsichtigtes Abgleiten des Schiebers 15 verhindert wird.

Fig. 2b zeigt den Klappenbeschlag im montierten Zustand. Hierbei sind die Teile 6a, 6b und 7 miteinander verbunden und bilden eine Einheit, die einerseits an der nicht dargestellten Klappe 3 und andererseits mit den Stellarmen 5a und 5b verbindbar ist (Fig. 2c). Über den Rasthebel 10b kann das Paket Lagerteil 7/Befestigungsteil 6b vom Grundkörper 6a gelöst werden. Durch die in Fig. 2a gezeigte Höhenverstellschraube 13, die an der Unterseite des Lagerteiles 7 zugänglich ist, kann das Lagerteil 7 relativ zum Befestigungsteil 6b (und damit zum Grundkörper 6a) in Höhenrichtung H verstellt werden. Durch die Seitenverstellschraube 11 kann das Befestigungsteil 6b in seiner seitlichen Ausrichtung B innerhalb des im Querschnitt U-förmigen Grundkörpers 6a verstellt werden. Schließlich kann durch die Neigungsverstellschraube 12 der Lagerteil 7 von der in Fig. 2b gezeigten 0°-Stellung relativ zum Paket Grundkörper 6a/Befestigungsteil 6b um den Winkelbetrag α geneigt werden.

Fig. 2c zeigt den Klappenbeschlag mit montierten Stellarmen 5a und 5b in einer perspektivischen Darstellung von hinten.

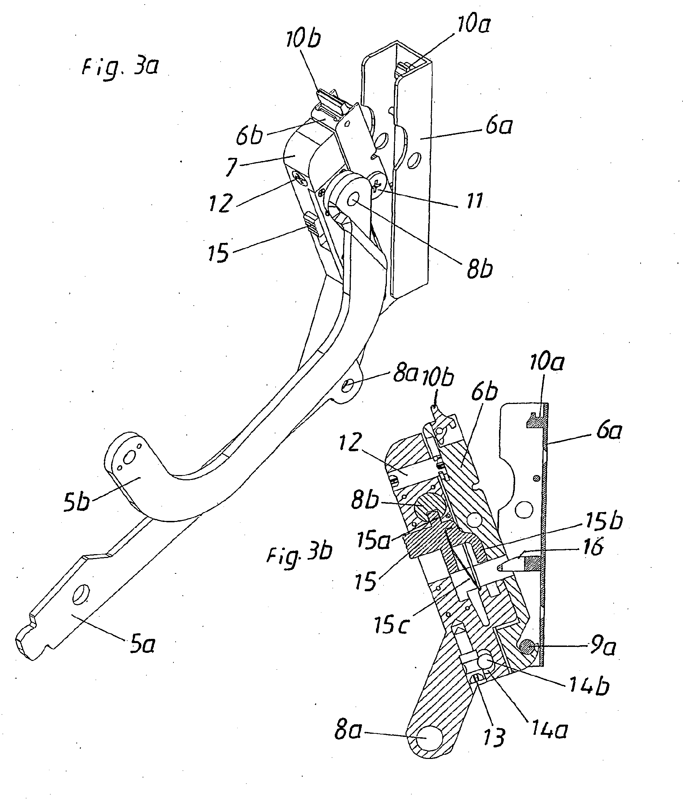

Fig. 3a zeigt eine perspektivische Vorderansicht des Klappenbeschlages mit montierten Stellarmen 5a und 5b, die über die Drehachsen 8a und 8b schwenkbar am Lagerteil 7 gelagert sind. Das klappenseitige Beschlagteil 6 umfasst den vormontierten Grundkörper 6a sowie den Befestigungsteil 6b, die über eine mechanische Rastverbindung 10a, 10b lösbar miteinander verbindbar sind. Fig. 3a und Fig. 3b zeigen den Beginn des Montageprozesses, wobei als erster Schritt der Befestigungsteil 6b in den Bolzen 9a des Grundkörpers 6a eingehängt wird. Nachstehend wird auf den Vertikalschnitt des Klappenbeschlages gemäß Fig. 3b Bezug genommen, in dem die Funktionsweise der Verdrehsicherung näher erläutert wird. Die Stellarme 5a, 5b weisen jeweils zwei Drehachsen 8a-8d auf, sodass diese durch deren korpusseitige Anlenkung mit dem klappenseitigen Beschlagteil 6 ein Viergelenk bilden. Der Stellarm 5b lagert an der Drehachse 8b, die gemäß Fig. 3b mit dem am Schieber 15 angeordneten Verriegelteil 15a in Eingriff steht, wodurch die Drehachse 8b des Stellarmes 5b in ihrer Schwenklage relativ zum klappenseitigen Beschlagteil 6a, 6b arretiert ist. Der Stellarm 5b lässt sich durch die Arretierung seiner Drehachse 8b weder in Bezug zum Möbelkorpus 2 noch in Bezug zum klappenseitigen Beschlagteil 6a, 6b verschwenken, wodurch eine einfache und rasch durchzuführende Montage der Klappe 3 ermöglicht wird. Die Blattfeder 15c stützt sich am Befestigungsteil 6b ab und verhindert ein unbeabsichtigtes Lösen des Schiebers 15. Zu erkennen ist zudem die Abhebesicherung 15b, die mit dem am Grundkörper 6a angeordneten Sicherungsbügel 16 in Eingriff bringbar ist, jedoch in der gezeigten Figur inaktiv ist. Die Neigungsverstellschraube 12 stützt sich mit ihrem Ende am Befestigungsteil 6b ab und bewirkt bei Verdrehung derselben eine Neigungsverstellung des Lagerteiles 7 in Bezug zum Befestigungsteil 6b. Durch die Höhenverstellschraube 13, in deren konkaven bzw. verjüngten Abschnitt der Bolzen 14b Aufnahme findet, kann der Lagerteil 7 entsprechend der Länge des Langlochs 14a verstellt werden, wodurch der Lagerteil 7 in Bezug zum Befestigungsteil 6b in der Höhe verstellbar ist.

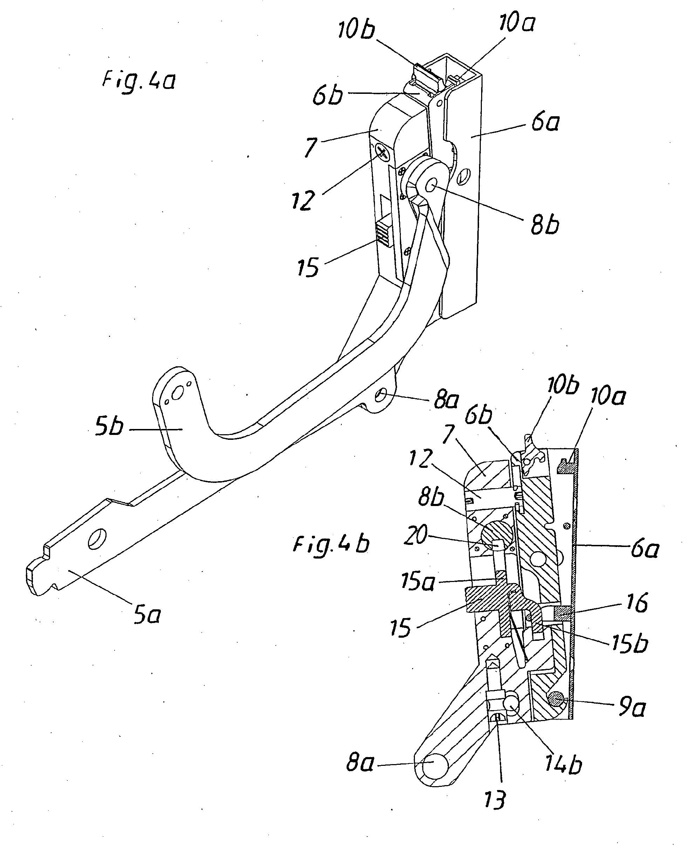

Fig. 4a zeigt wieder die perspektivische Vorderansicht des Klappenbeschlages aus Fig. 3a, mit dem Unterschied, dass das Paket Lagerteil 7/Befestigungsteil 6b näher zum Grundkörper 6a hin verschwenkt wurde und dass der Schieber 15 in seine zweite Betriebsstellung verschoben wurde. Aus dem Vertikalschnitt gemäß Fig. 4b geht hervor, dass durch die erfolgte Verschiebung des Schiebers 15 der Verriegelteil 15a von der Drehachse 8b außer Eingriff gebracht wurde, sodass nunmehr alle Drehachsen 8a-8d und damit die Stellarme 5a, 5b frei beweglich sind. Zu erkennen ist zudem die Ausnehmung 20, die radial zur Drehachse 8b verläuft und in die der Verriegelteil 15a des Schiebers 15 einbringbar ist. Der Schieber 15 erfüllt überdies eine Doppelfunktion, da nunmehr die Abhebesicherung 15b mit dem Sicherungsbügel 16 in Eingriff ist. Falls unbeabsichtigt die Rastverbindung 10a, 10b gelöst wird, kann sich das Paket Lagerteil 7/Befestigungsteil 6b nur teilweise vom vormontierten Grundkörper 6a lösen und ist daher unverlierbar gegen ein Abstürzen der Klappe 3 gesichert.

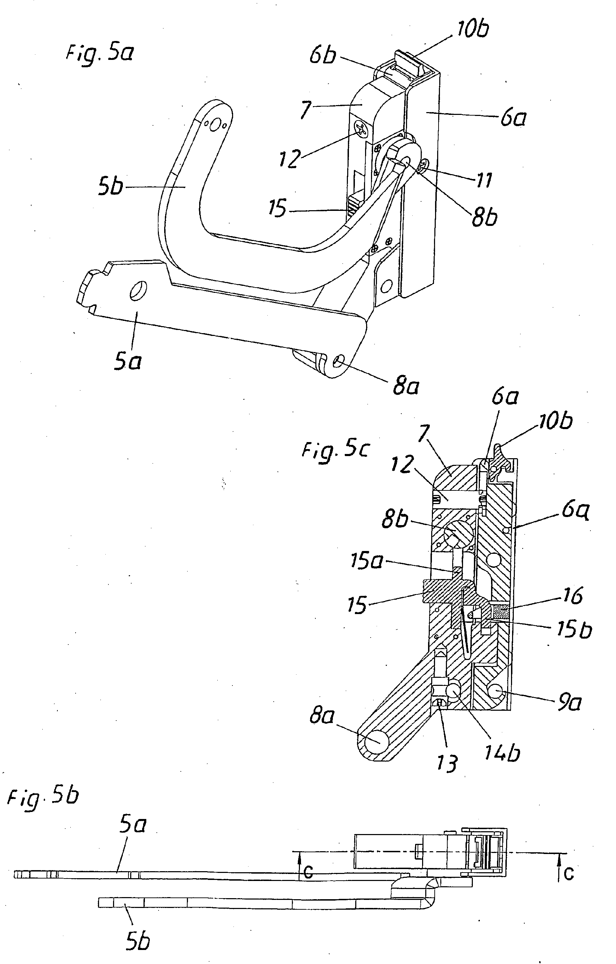

Fig. 5a zeigt die vollständig verrastete Stellung des Paketes Lagerteil 7/Befestigungsteil 6b mit dem an der Klappe 3 vormontierten Grundkörper 6a. Fig. 5b zeigt eine Draufsicht auf den Klappenbeschlag mit seinen Stellarmen 5a und 5b, während Fig. 5c den Vertikalschnitt entlang der Linie C-C aus Fig. 5b zeigt. Gemäß Fig. 5c ist der Rasthebel 10b eingerastet, die Drehachse 8b ist vom Verriegelteil 15a freigegeben und die Abhebesicherung 15b des Schiebers 15 ist mit dem Sicherungsbügel 16 in Eingriff, sodass die Klappe 3 einerseits durch die Rastverbindung 10a, 10b und andererseits durch die Abhebesicherung 15b gegen Absturz doppelt gesichert ist. Diese gezeigte Stellung des Schiebers 15 entspricht der Gebrauchslage während dem Normalbetrieb des Klappenbeschlages.

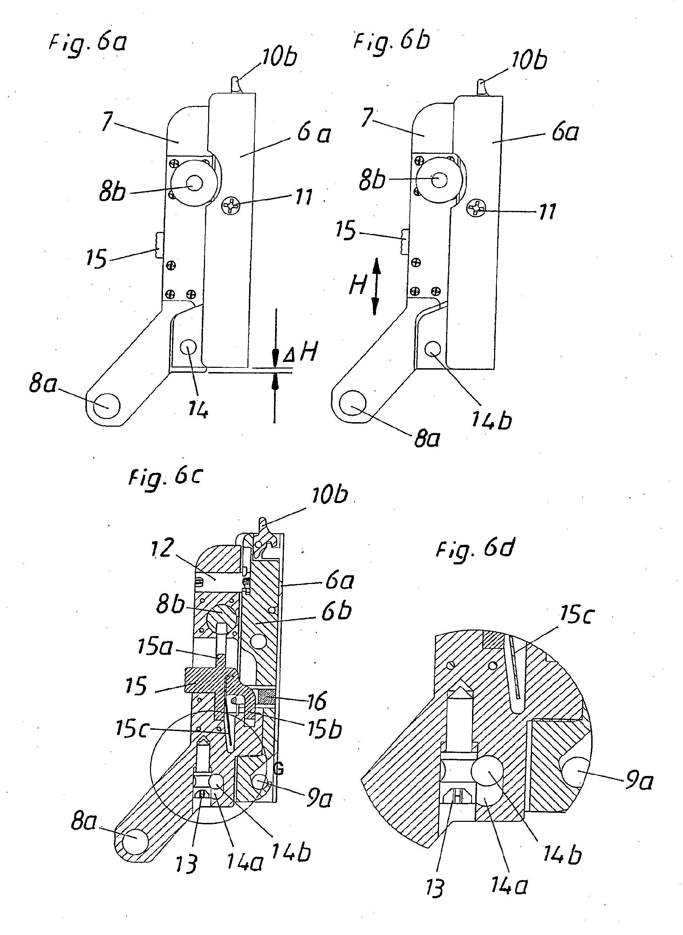

Fig. 6a bis 6d zeigen verschiedene Ansichten des Klappenbeschlages, in denen der Vorgang der Höhenverstellung erläutert wird. Fig. 6a zeigt eine Seitenansicht des Grundkörpers 6a, der an einer nicht dargestellten Klappe 3 vormontiert ist. Über eine mechanische Rastverbindung (Rasthebel 10b) wurde das Paket Lagerteil 7/Befestigungsteil 6b auf den Grundkörper 6a angeklippt. Durch die in Fig. 6c gezeigte Höhenverstellschraube 13 kann der Lagerteil 7 relativ zum Befestigungsteil 6b um die Differenzhöhe ΔH verstellt werden. Fig. 6b zeigt den Klappenbeschlag aus Fig. 6a, mit dem in der Höhe H verstellten Lagerteil 7. Fig. 6c zeigt einen Vertikalschnitt durch den Klappenbeschlag, bei dem die zur Höhenverstellung vorgesehene Höhenverstellschraube 13 dargestellt ist. Diese Höhenverstellschraube 13 stützt sich mit ihrem verjüngten Abschnitt am Bolzen 14b ab. Der Bolzen 14b ist am Befestigungsteil 6b ortsfest gelagert, bei Betätigung der Höhenverstellschraube 13 wird daher das Lagerteil 7 höhenverstellt, wobei die maximale Höhenverstellung der Ausdehnung des Langlochs 14a entspricht. Fig. 6d zeigt die Detailansicht G des Kreises aus Fig. 6c.

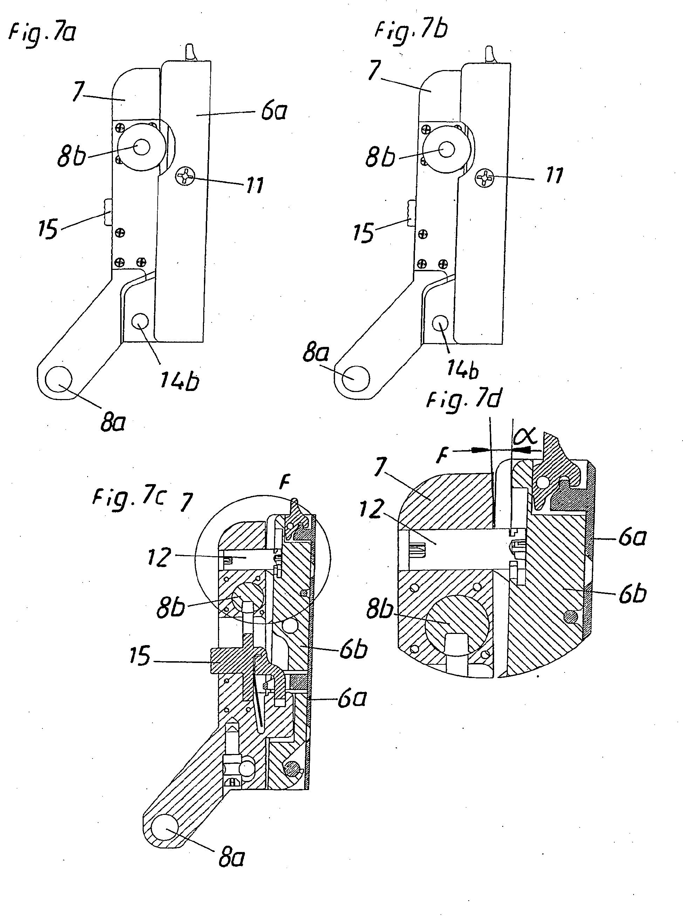

Fig. 7a bis 7d zeigen verschiedene Ansichten des Klappenbeschlages, in denen der Vorgang der Neigungsverstellung erläutert wird. Fig. 7a zeigt eine Seitenansicht des Klappenbeschlages mit leicht geneigtem Lagerteil 7 in Bezug zum Paket Grundkörper 6a/Befestigungsteil 6b, während Fig. 7b die parallele Ausrichtung (0° Neigung) des Lagerteiles 7 zeigt. Fig. 7c zeigt einen Vertikalschnitt durch den Klappenbeschlag, wobei die Neigung α durch Eindrehen der Neigungsverstellschraube 12, die einerseits mit einem Innengewinde des Lagerteiles 7 zusammenwirkt und andererseits an ihrem hinteren Ende am Befestigungsteil 6b abstützt, veränderbar ist. Durch Drehen der Neigungsverstellschraube 12 im Uhrzeigersinn wird der Neigungswinkel α vergrößert, bei Drehung im Gegenuhrzeigersinn entsprechend verringert. Fig. 7d zeigt die Detailansicht F aus Fig. 7c mit den um den Neigungswinkel α verstellten Lagerteil 7.

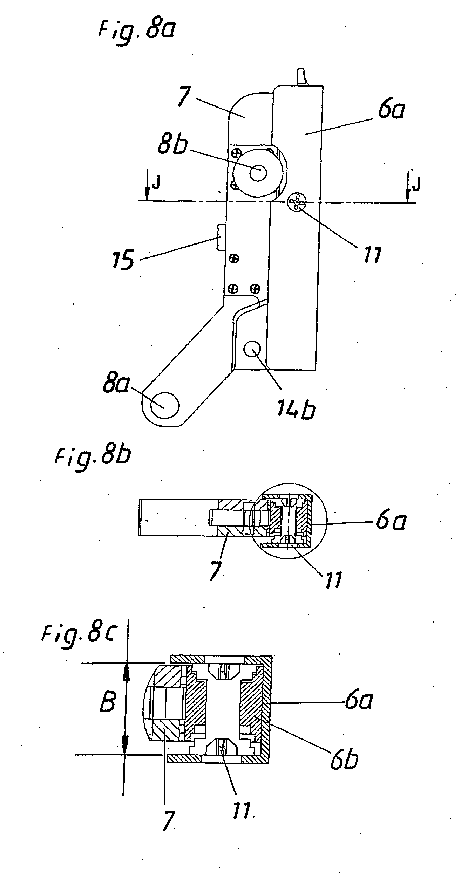

Fig. 8a bis 8c zeigen verschiedene Ansichten des Klappenbeschlages, in denen der Vorgang der seitlichen Ausrichtung bzw. Querverstellung des Paketes Lagerteil 7/Befestigungsteil 6b relativ zum Grundkörper 6a erläutert wird. Fig. 8a zeigt eine Seitenansicht des Klappenbeschlages mit der Seitenverstellschraube 11. Fig. 8b zeigt einen Schnitt in Richtung der Pfeile J-J aus Fig. 8a, während Fig. 8c die vergrößerte Detaildarstellung aus Fig. 8b zeigt. Die Seitenverstellschraube 11 lagert zwischen den beiden Schenkeln des U-förmigen Grundkörpers 6a und ist dort ortsfest aber drehbar gelagert. Die Seitenverstellschraube 11 weist ein Außengewinde auf, welches mit einem Innengewinde des Befestigungsteil 6b in Eingriff steht. Bei Verdrehung der Seitenverstellschraube 11 lässt sich somit der Befestigungsteil 6b mit seinem daran befestigten Lagerteil 7 in der Breite B verstellen, wobei die maximale Breitenverstellung der Beabstandung der beiden Schenkel des U-förmigen Grundkörpers 6a entspricht.

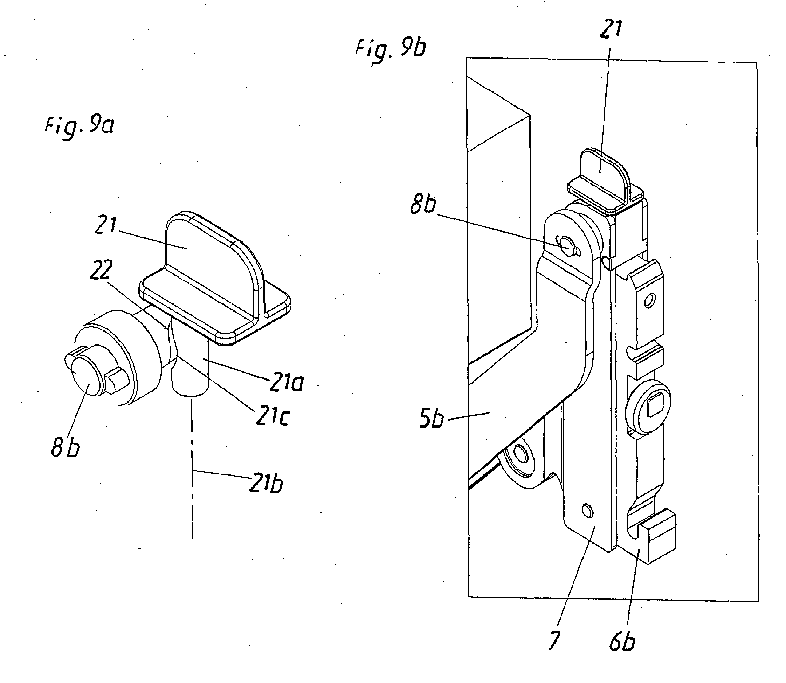

Fig. 9a und Fig. 9b zeigen ein alternatives Ausführungsbeispiel der Verdrehsicherung des Stellarmes 5b. Anstelle des in den Fig. 1 bis 8 gezeigten Schiebers 15 ist ein drehbarer Teil 21 vorgesehen, der in einer ersten Drehstellung die Drehachse 8b in ihrer Schwenklage arretiert und in einer weiteren Drehstellung eine Schwenkbewegung der Drehachse 8b und damit auch ein Verschwenken des Stellarmes 5b erlaubt. Der drehbare Teil 21 umfasst ein Verriegelteil in Form eines Zylinders 21 a mit einer Zylinderachse 21 b. Der Zylinder 21 a weist eine zylindrische Ausnehmung 21 c auf, die quer zur Zylinderachse 21 b verläuft. Der zylindrische Teil der Drehachse 8b weist ebenfalls eine zylindrische Vertiefung 22 auf, die quer zur Längserstreckung der Drehachse 8b verläuft. In der in Fig. 9a gezeigten Drehstellung des drehbaren Teiles 21 ist eine Verdrehbewegung der Drehachse 8b möglich.

Fig. 9b zeigt diese alternative Verdrehsicherung am klappenseitigen Möbelbeschlag mit dem Paket Lagerteil 7/Befestigungsteil 6b. Der Stellarm 5b lagert schwenkbar an der Drehachse 8b und wird in der gezeigten Drehstellung des drehbaren Teiles 21 in seiner Schwenklage nicht arretiert.

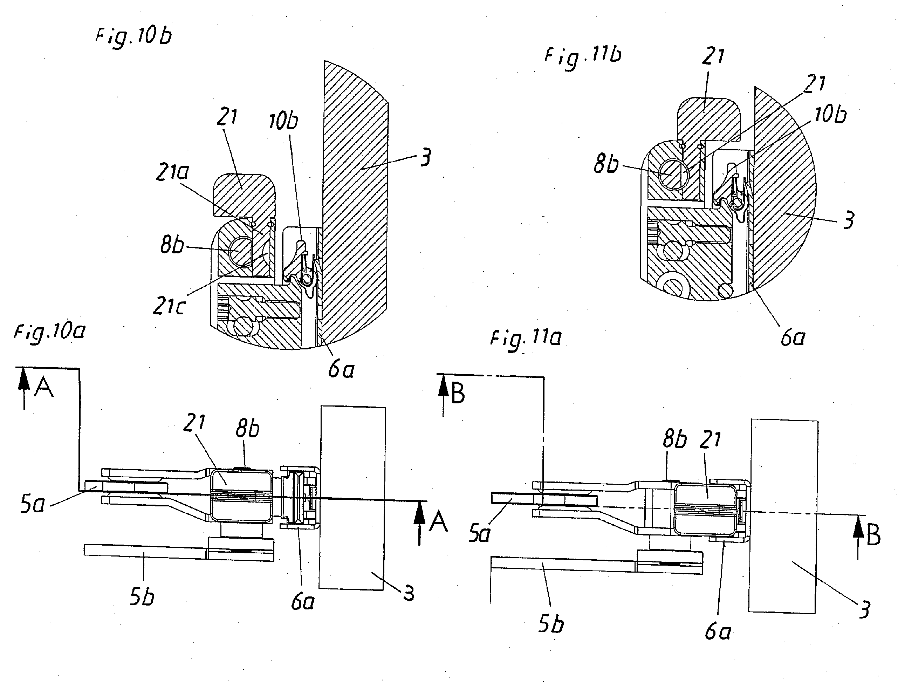

Fig. 10a zeigt eine Draufsicht auf den an der Klappe 3 montierten Klappenbeschlag mit der in Fig. 9a und 9b gezeigten Verdrehsicherung. Der Grundkörper 6a ist an der Klappe 3 montiert und mit dem Paket Lagerteil 7/Befestigungsteil 6b - an dem die beiden Stellarme 5a und 5b lagern - lösbar verbunden. Fig. 10b zeigt die Schnittdarstellung in Richtung der Pfeile A-A aus Fig. 10a. Der drehbare Teil 21 befindet sich in der Drehstellung, in der er die Drehachse 8b in ihrer Schwenklage fixiert, da die Ausnehmung 21 c des drehbaren Teiles 21 von der Schwenkachse 8b abgewandt ist. Eine Verdrehung der Drehachse 8b des Stellarmes 5b ist in der in Fig. 10a und Fig. 10b gezeigten Stellung des drehbaren Teiles 21 nicht möglich.

Fig. 11a und Fig. 11b zeigen analog die Drehstellung des drehbaren Teiles 21, in der eine Verdrehbewegung der Drehachse 8b des Stellarmes 5b möglich ist. Durch Drehung des drehbaren Teils 21 um 180° wurde die zylindrische Vertiefung 21 zur Drehachse 8b hin verdreht, sodass die korrespondierenden Vertiefungen 21 c und 22 (Fig. 9a) eine Drehbewegung des Stellarmes 5b erlauben. Ein besonderer Vorteil bei der Ausführungsform mit diesem drehbaren Teil 21 liegt darin, dass aufgrund der in Fig. 11a gezeigten Drehstellung des drehbaren Teiles 21 der Zugriff auf den Rasthebel 10b nicht möglich ist. Der drehbare Teil 21 erfüllt neben der Hemmung der Verschwenkbewegung eine weitere Funktion, nämlich, dass man unbeabsichtigt mit dem Schraubendreher zum Rasthebel 10b gelangt. Im Gegensatz zu der in Fig. 4b gezeigten aktiven Abhebesicherung 15b, die mit dem Sicherungsbügel 16 in Eingriff bringbar ist, wird durch die Drehstellung des drehbaren Teiles eine passive Abhebesicherung realisiert.

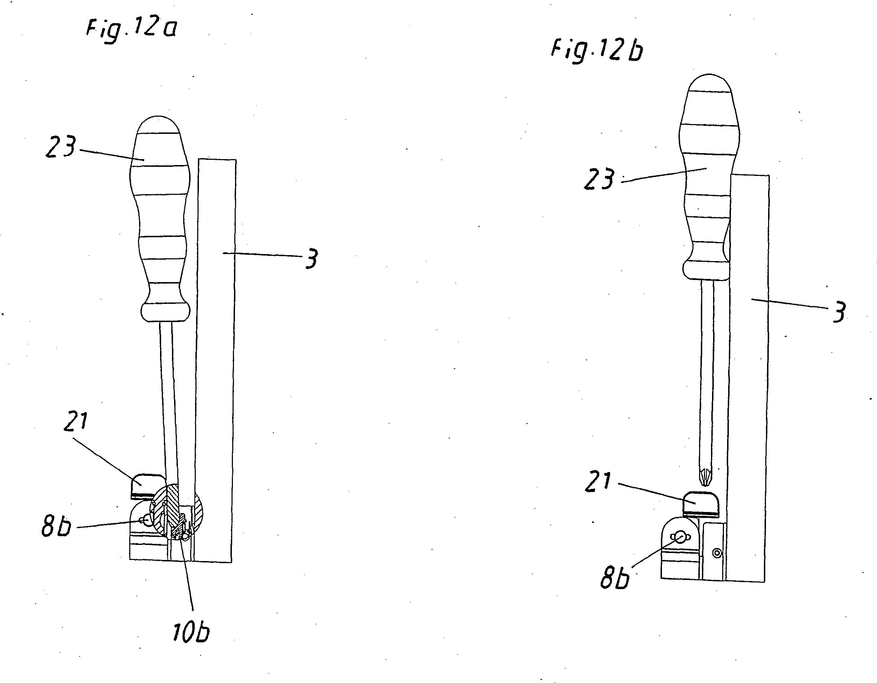

Fig. 12a und Fig. 12b zeigen diese passive Abhebesicherung je nach Drehstellung des drehbaren Teiles 21. In der in Fig. 12a gezeigten Stellung des drehbaren Teiles 21 kann der Rasthebel 10b mit einem Schraubendreher 23 betätigt werden, während in der in Fig. 12b gezeigten Drehstellung des drehbaren Teiles 21 ein Einbringen des Schraubendrehers 23 nicht möglich ist.

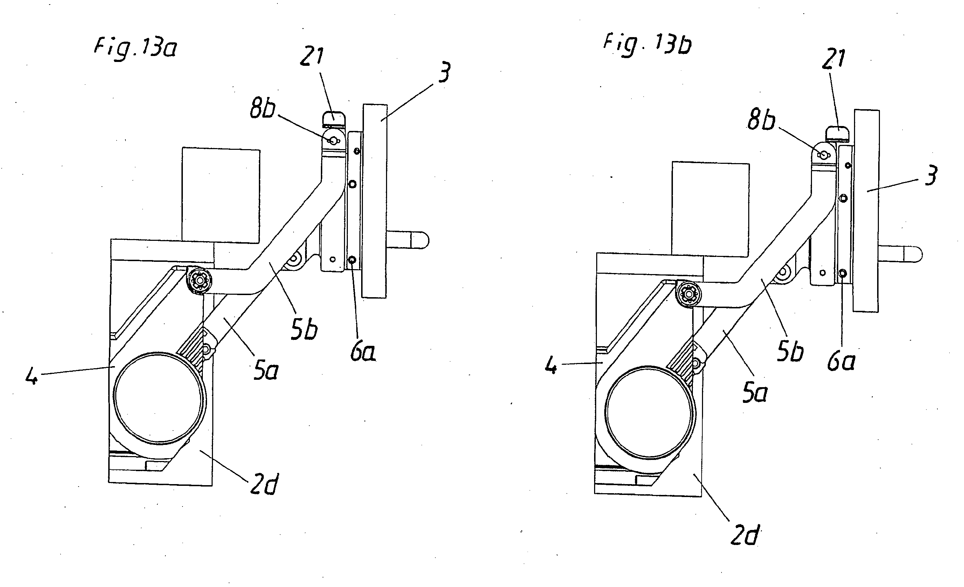

Fig. 13a und Fig. 13b zeigen die Ausführungsform des drehbaren Teiles 21 gemäß den Fig. 9 bis 12, der Teil der Verdrehsicherung für den Stellarm 5b ist. An der Seitenwand 2d eines schrankförmigen Möbels ist ein Stellmechanismus 4 mit zwei Stellarmen 5a und 5b montiert. Fig. 13a zeigt die vollständige Offenstellung der Klappe 3, wobei die Drehachse 8b durch die Drehstellung des drehbaren Teiles 21 in ihrer Schwenklage arretiert ist und auch ein Einbringen eines Schraubendrehers in den nicht dargestellten Rasthebel 10b im Grundkörper 6a von oben her möglich ist. Fig. 13b zeigt hingegen die um 180° verdrehte Stellung des drehbaren Teiles 21, wobei in dieser Stellung die Drehachse 8b des Stellarmes 5b nicht arretiert ist. Gemäß dieser Figur ist eine Verschwenkung der Stellarme 5a und 5b möglich, der Zutritt zum Rasthebel 10b der lösbaren Befestigungsvorrichtung dagegen verwehrt.

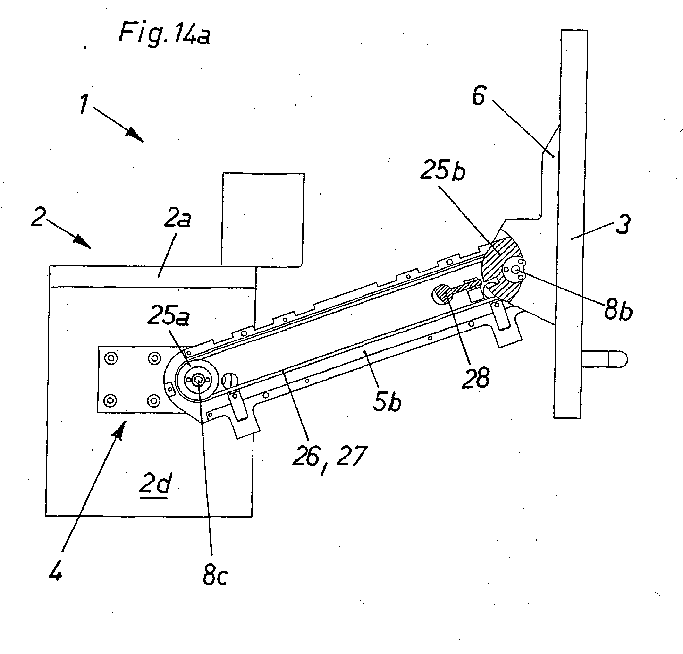

Fig. 14a zeigt schematisch eine Variante der Erfindung. Das Möbel 1 umfasst einen Schrankkorpus 2 mit einem Schrankdeckel 2a und einer Seitenwand 2d. Auf jeder Seitenwand 2d des Schrankkorpus 2 ist ein Stellmechanismus 4 angeordnet, der lediglich einen einzigen Stellarm 5b mit zwei Drehachsen 8b und 8c zum Bewegen der Möbelklappe 3 aufweist. Koaxial zur Drehachse 8c ist ein drehfest gelagerter Umlenkteil 25a (z.B. ein drehfestes Zahnrad) angeordnet, wobei ein Seil 26, vorzugsweise ein Zahnriemen 27, über einen weiteren Umlenkteil 25b (vorzugsweise ein weiteres Zahnrad) geführt ist. Der weitere Umlenkteil 25b hingegen ist drehbar am freien Ende des Stellarmes 5b gelagert und ist jedoch drehfest mit dem klappenseitigen Beschlagteil 6 verbunden. Über den Zahnriemen 27 wird eine Zwangsführung des zwischen dem drehfesten Umlenkteil 25a und dem drehbaren Umlenkteil 25b (und damit des klappenseitigen Beschlagteiles 6 bzw. der Möbelklappe 3) ermöglicht, sodass die Möbelklappe 3 während ihrer Bewegung ständig parallel zur Stirnseite des Möbelkorpus 2 geführt wird. Zu erkennen ist ein am freien Ende des Stellarmes 5b schematisch dargestellter Schieber 28, der in eine korrespondierende Ausnehmung des Umlenkteiles 25b in Eingriff bringbar ist. Der Schieber 28 ist Teil der Verdrehsicherung, die jedoch in der gezeigten Figur deaktiviert ist, d.h. die Möbelklappe 3 ist zwischen einer Schließ- und einer Offenstellung im Wesentlichen frei bewegbar.

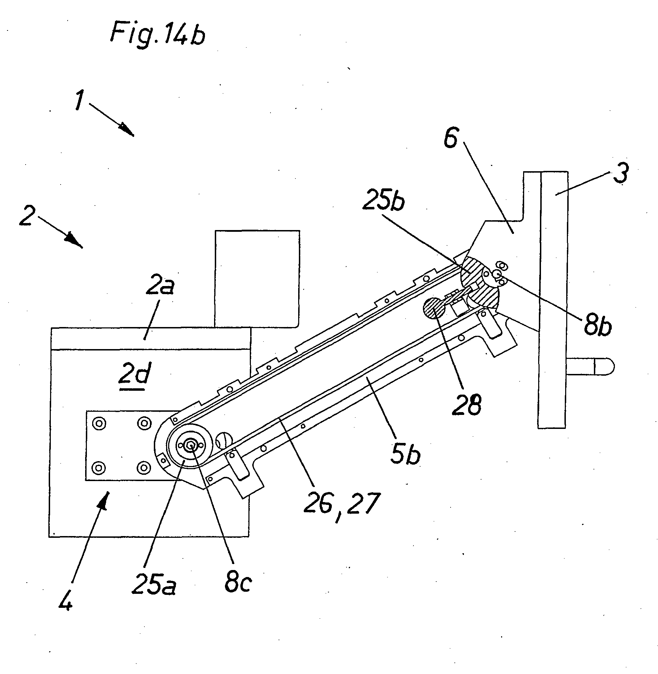

Fig. 14b zeigt hingegen die Stellung des Schiebers 28, in welcher dieser in die Ausnehmung des Umlenkteiles 25b eingreift. Somit ist neben dem drehfesten Umlenkteil 25a am Schrankkorpus auch der weitere Umlenkteil 25b vorläufig nicht drehbar und der Zahnriemen 27 hält somit den Stellarm 5b in seiner vollständigen Offenstellung, sodass die Möbelklappe 3 in komfortabler Weise am - relativ zum Stellarm 5b arretierten - klappenseitigen Beschlagteil 6 zu montieren ist, ohne dass zudem die Gefahr besteht, dass der Stellarm 5b in das Innere des Möbelkorpus abgleiten bzw. durch eine beaufschlagende Federvorrichtung wieder in die Höhe schnellen und dabei Verletzungen verursachen kann.

Die vorliegende Erfindung beschränkt sich nicht auf die gezeigten Ausführungsbeispiele sondern umfasst bzw. erstreckt sich auf alle Varianten und technischen Äquivalente, die in die Reichweite der nachfolgenden Ansprüche fallen können. Auch sind die in der Beschreibung gewählten Lageangaben wie z.B. oben, unten, seitlich usw. auf die übliche Einbaulage des Klappenbeschlages bzw. auf die unmittelbar beschriebene sowie dargestellte Figur bezogen und sind bei einer Lageänderung sinngemäß auf die neue Lage zu übertragen. Auch kann die erfindungsgemäße Verdrehsicherung dahingehend realisiert werden, dass eine die Drehachse 8b umgreifende Klemmvorrichtung vorgesehen wird. Es liegt auch im Rahmen der Erfindung, hierfür Klemmexzenter o. Ä. anzuordnen.

## Patentansprüche

1. Klappenbeschlag zum schwenkbaren Befestigen einer Möbelklappe (3) an einem Schrankkorpus (2), mit mindestens einem Stellarm (5b), der zum Bewegen der Möbelklappe (3) vorgesehen ist, und mit mindestens einem klappenseitigen Beschlagteil (6), der mit dem Stellarm (5b) verbindbar ist, wobei der klappenseitige Beschlagteil (6) eine Verdrehsicherung zum zeitweiligen Fixieren der Schwenklage des mindestens einen Stellarmes (5b), vorzugsweise in dessen vollständiger Offenstellung, aufweist, wobei die Verdrehsicherung in einer ersten Betriebsstellung den mindestens einen Stellarm (5b) in seiner Schwenklage relativ zum klappenseitigen Beschlagteil (6) arretiert und in einer zweiten Betriebsstellung eine Schwenkbewegung des Stellarmes (5b) erlaubt, **dadurch gekennzeichnet, dass** die Verdrehsicherung ein Verriegelteil (15a, 21a, 28) aufweist, der in der arretierenden ersten Betriebsstellung in einer Ausnehmung (20, 22) eingreift, welche an einem um eine Drehachse (8b) des Stellarmes (5b) drehbar gelagerten Lagerbolzen (8c) oder an einem um eine Drehachse (8b) des Stellarmes (5b) drehbar gelagerten Umlenkteil (25b) angeordnet ist.

2. Klappenbeschlag nach Anspruch 1, **dadurch gekennzeichnet, dass** auf jeder Seitenwand (2d) des Schrankkorpus (2) wenigstens zwei Stellarme (5a, 5b) mit jeweils zwei Drehachsen (8a-8d) zum Bewegen der Möbelklappe) (3) vorgesehen sind, wobei durch die Verdrehsicherung wenigstens eine Drehachse (8a-8d) der Stellarme (5a, 5b) arretierbar ist.

3. Klappenbeschlag nach Anspruch 2, **dadurch gekennzeichnet, dass** die wenigstens zwei Stellarme (5a, 5b) mit dem klappenseitigen Beschlagteil (6) ein Viergelenk bilden.

4. Klappenbeschlag nach Anspruch 1, **dadurch gekennzeichnet, dass** auf jeder Seitenwand (2d) des Schrankkorpus (2) lediglich ein Steilarm (5b) mit zwei Drehachsen (8b, 8c) zum Bewegen der Möbelklappe (3) vorgesehen ist, wobei mindestens ein zusätzliches Seil (26), vorzugsweise ein Zahnriemen (27), zwischen einem drehfest am Schrankkorpus (2) gelagerten Umlenkteil (25a) und dem Umlenkteil (25b) geführt ist, der drehbar am freien Ende des Stellarmes (5b) gelagert ist und der drehfest mit dem klappenseitigen Beschlagteil (6) verbindbar ist, wobei durch die Verdrehsicherung der Umlenkteil (25b) relativ zum Stellarm (5b) arretierbar ist.

5. Klappenbeschlag nach Anspruch 4, **dadurch gekennzeichnet, dass** die Verdrehsicherung einen am Stellarm (5b) angeordneten Schieber (28) aufweist, der mit dem weiteren Umlenkteil (25b) des klappenseitigen Beschlagteiles (6) in Eingriff bringbar ist.

6. Klappenbeschlag nach einem der Ansprüche 1 bis 5, **dadurch gekennzeichnet, dass** die Ausnehmung (20) radial zur Drehachse (8b) des Stellarmes (5b) verläuft.

7. Klappenbeschlag nach einem der Ansprüche 1 bis 5, **dadurch gekennzeichnet, dass** die Ausnehmung (22) quer zur Drehachse (8b) des Stellarmes (5b) verläuft.

8. Klappenbeschlag nach einem der Ansprüche 1 bis 7, **dadurch gekennzeichnet, dass** der Verriegelteil (15a) von einem Schieber (15) gebildet ist.

9. Klappenbeschlag nach einem der Ansprüche 1 bis 7, **dadurch gekennzeichnet, dass** der Verriegelteil (21 a) von einem drehbaren Teil (21) gebildet ist.

10. Klappenbeschlag nach Anspruch 9, **dadurch gekennzeichnet, dass** die Ausnehmung (22) eine zylindrische Vertiefung ist.

11. Klappenbeschlag nach Anspruch 9 oder 10, **dadurch gekennzeichnet, dass** der drehbare Teil (21) einen Zylinder (21a) mit einer zylindrischen Ausnehmung (21c) aufweist, die quer zur Zylinderachse (21b) verläuft.

12. Klappenbeschlag nach einem der Ansprüche 1 bis 11, **dadurch gekennzeichnet, dass** der klappenseitige Beschlagteil (6) einen an der Klappe (3) vormontierten Grundkörper (6a) und einen mit dem Stellarm (5a, 5b) verbundenen Befestigungsteil (6b) aufweist.

13. Klappenbeschlag nach Anspruch 12, **dadurch gekennzeichnet, dass** der Befestigungsteil (6b) mit dem Grundkörper (6a) durch eine lösbare Befestigungsvorrichtung (10a, 10b), vorzugsweise eine mechanische Rastverbindung, verbindbar ist.

14. Klappenbeschlag nach Anspruch 13, **dadurch gekennzeichnet, dass** die mechanische Rastverbindung derart ausgebildet ist, dass der Befestigungsteil (6b) in den Grundkörper (6a) einhängbar und anschließend durch Aufschwenken mit diesem verrastbar ist.

15. Klappenbeschlag nach Anspruch 13 oder 14, **dadurch gekennzeichnet, dass** der Befestigungsteil (6b) mit dem Grundkörper (6a) zusätzlich zur lösbaren Befestigungsvorrichtung (10a, 10b) durch eine gesonderte Abhebesicherung (15b) gesichert ist.

16. Klappenbeschlag nach Anspruch 15, **dadurch gekennzeichnet, dass** die Abhebesicherung (15b) einen vorzugsweise federbeaufschlagten, Schieber (15) umfasst, der in einer Betriebsstellung mit einem am Grundkörper (6a) oder am Befestigungsteil (6b) angeordneten oder ausgebildeten Sicherungsbügel (16) in Eingriff bringbar ist.

17. Klappenbeschlag nach Anspruch 16, **dadurch gekennzeichnet, dass** die Verdrehsicherung (15a) vom selben Schieber (15) wie die Abhebesicherung (15b) gebildet ist.

18. Klappenbeschlag nach einem der Ansprüche 1 bis 17, **dadurch gekennzeichnet, dass** die relative Lage des Stellarmes (5a, 5b) in Bezug zum klappenseitigen Beschlagteil (6) durch wenigstens eine Einstellvorrichtung (11, 12, 13) veränderbar ist.

19. Klappenbeschlag nach Anspruch 18, **dadurch gekennzeichnet, dass** durch die wenigstens eine Einstellvorrichtung (11, 12, 13) der Stellarm (5a, 5b) in Bezug zum klappenseitigen Beschlagteil (6) in seiner Höhe (H) und/oder in seiner Neigung (α) und/oder in seiner seitlichen Ausrichtung (B) verstellbar ist.

20. Klappenbeschlag nach Anspruch 18 oder 19, **dadurch gekennzeichnet, dass** für die Verstellung der Höhe (H), der Neigung (α) und der seitlichen Ausrichtung (B) jeweils eine gesonderte Einstellvorrichtung (11, 12, 13) vorgesehen ist.

21. Klappenbeschlag nach einem der Ansprüche 18 bis 20, **dadurch gekennzeichnet, dass** die wenigstens eine Einstellvorrichtung (11, 12, 13) mindestens eine Gewindeschraube aufweist, die manuell oder durch ein Verstellelement, vorzugsweise einen Schraubendreher, betätigbar ist.

22. Klappenbeschlag nach einem der Ansprüche 1 bis 21, **dadurch gekennzeichnet, dass** am klappenseitigen Beschlagteil zwei Stellarme (5a, 5b) schwenkbar gelagert sind.

## Claims

1. A flap fitting for a swivellable attachment of a furniture flap (3) to a cabinet body (2), with at least one actuating arm (5b) for moving the furniture flap (3), and with at least one flap side fitting part (6) which can be connected to the actuating arm (5b), wherein the flap side fitting part (6) comprises an anti-twist device for temporary fixing of a swivel position of the at least one actuating arm (5b), preferably in its completely open position, wherein in a first operating position, the anti-twist device locks the at least one actuating arm (5b) in its swivel position relative to the flap side fitting part (6), and in a second operating position allows a swivel movement of the actuating arm (5b), **characterized in that** the anti-twist device has a locking part (15a, 21a, 28) which, in the first operating position when locking, engages into a recess (20, 22) which is arranged on a bearing bolt (8c) being pivotally arranged about a pivot axis (8b) of the actuating arm (5b) or on a deflection part (25b) being pivotally arranged about a pivoting axis (8b) of the actuating arm (5b)

2. The flap fitting according to claim 1, **characterized in that** on every side wall (2d) of the cabinet body (2), at least two actuating arms (5a, 5b) each with two pivot axes (8a-8d) are provided for moving the furniture flap (3), wherein at least one pivot axis (8a-8d) of the actuating arms (5a, 5b) can be locked by the anti-twist device.

3. The flap fitting according to claim 2, **characterized in that** the at least two actuating arms (5a, 5b), together with the flap-side fitting part (6), form a four-bar mechanism.

4. The flap fitting according to claim 1, **characterized in that** on every side wall (2d) of the cabinet body (2c), only one actuating arm (5b) with two pivot axes (8b, 8c) is provided for moving the furniture flap (3), wherein at least one additional cord (26), preferably a toothed belt (27), is guided between a guiding part (25a) being arranged rotation-resistant at the cabinet body (2) and the deflection part (25b) which is rotatably arranged at the free end of the actuating arm (5b) and which can be connected rotation-resistant to the flap-side fitting part (6), wherein the deflection part (25b) can be locked relative to the actuating arm (5b) by the anti-twist device (5b).

5. The flap fitting according to claim 4, **characterized in that** the anti-twist device comprises a slider (28) arranged on the actuating arm (5b), the slider (28) can be brought into engagement with the deflection part (25b) of of the flap-side fitting part (6).

6. The flap fitting according to one of the claims 1 to 5, **characterized in that** the recess (20) runs radially to the pivot axis (8b) of the actuating arm (5b).

7. The flap fitting according to one of the claims 1 to 6, **characterized in that** the recess (22) runs transversely to the pivot axis (8b) of the actuating arm (5b).

8. The flap fitting according to one of the claims 1 to 7, **characterized in that** the locking part (15a) is formed from a slider (15).

9. The flap fitting according to one of the claims 1 to 7, **characterized in that** the locking part (21 a) is formed by a rotatable part (21).

10. The flap fitting according to claim 9, **characterized in that** the recess (22) of the pivot axis (8b) is a cylindrical depression.

11. The flap fitting according to claim 9 or 10, **characterized in that** the rotatable part (21) has a cylinder (21 a) with a cylindrical recess (21c) which runs transversely to the cylinder axis (21 b).

12. The flap fitting according to one of the claims 1 to 11, **characterized in that** the flap side fitting part (6) has a base (6a) pre-fitted on the flap (3) and an attachment part (6b) connected to the actuating arm (5a, 5b).

13. The flap fitting according to claim 12, **characterized in that** the attachment part (6b) can be connected to the base (6a) by a releasable attachment device (10a, 10b), preferably a mechanical latching connection.

14. The flap fitting according to claim 13, **characterized in that** the mechanical latching connection is formed such that the attachment part (6b) can be inserted into the base (6a) and then locked to this by being swivelled.

15. The flap fitting according to claim 13 or 14, **characterized in that** the attachment part (6b) is secured to the base (6a), additionally to the releasable attachment device (10a, 10b), by a separate anti-lifting device (15b).

16. The flap fitting according to claim 15, **characterized in that** the anti-lifting device (15b) comprises a, preferably spring-loaded, slider (15) which in one operating position can be brought into engagement with a safety catch (16) attached to or formed on the base (6a) or the attachment part (6b).

17. The flap fitting according to claim 16, **characterized in that** the anti-twist device (15a) is formed from the same slider (15) as the anti-lifting device (15b).

18. The flap fitting according to one of claims 1 to 17, **characterized in that** the relative position of the actuating arm (5a, 5b) in relation to the flap-side fitting part (6) can be adjusted by at least one adjustment device (11, 12, 13).

19. The flap fitting according to claim 18, **characterized in that** the height (H) of the actuating arm (5a, 5b) and/or its inclination (α) and/or the lateral alignment (B) in relation to the flap-side fitting part (6) can be adjusted by the at least one adjustment device (11, 12, 13).

20. The flap fitting according to claim 18 or 19, **characterized in that** in each case a separate adjustment device (11, 12, 13) is provided to adjust the height (H), the inclination (α) and the lateral alignment (B).

21. The flap fitting according to one of the claims 18 to 20, **characterized in that** the at least one adjustment device (11, 12, 13) has at least one threaded screw which can be actuated manually or by a setting element, preferably a screwdriver.

22. The flap fitting according to one of the claims 1 to 21, **characterized in that** two actuating arms (5a, 5b) are rotatably arranged at the flap-side fitting part (6).

## Revendications

1. Ferrure pour abattant destinée à la fixation pivotante d'un abattant (3) de meuble sur un corps d'armoire (2), comportant au moins un bras de positionnement (5b) qui est prévu pour le mouvement de l'abattant (3) de meuble, et comportant au moins un élément de ferrure (6) du côté abattant, qui est destiné à être relié au bras de positionnement (5b), ledit élément de ferrure (6) du côté abattant comportant un système anti-rotation pour immobiliser temporairement ledit au moins un bras de positionnement (5b) dans une position de pivotement, de préférence dans la position complètement ouverte de celui-ci, le système anti-rotation immobilisant, dans une première position de service, ledit au moins un bras de positionnement (5b) dans sa position de pivotement par rapport à un élément de ferrure (6) du côté abattant et permettant, dans une deuxième position de service, un mouvement de pivotement du bras de positionnement (5b), **caractérisée en ce que** le système anti-rotation comporte un élément de verrouillage (15a, 21a, 28) qui, dans la première position de service immobilisante, s'engage dans un évidement (20, 22) disposé sur un boulon d'appui (8c) monté rotatif autour d'un axe de rotation (8b) du bras de positionnement (5b) ou sur un élément de renvoi (25b) monté rotatif autour d'un axe de rotation (8b) du bras de positionnement (5b).

2. Ferrure pour abattant selon la revendication 1, **caractérisée en ce que** sur chaque paroi latérale (2d) du corps d'armoire (2) sont prévus au moins deux bras de positionnement (5a, 5b) ayant chacun deux axes de rotation (8a-8d) pour le mouvement de l'abattant de meuble (3), au moins un axe de rotation (8a-8d) des bras de positionnement (5a, 5b) pouvant être bloqué par le système anti-rotation.

3. Ferrure pour abattant selon la revendication 2, **caractérisée en ce que** lesdits au moins deux bras de positionnement (5a, 5b) forment avec l'élément de ferrure (6) du côté abattant un quadrilatère articulé.

4. Ferrure pour abattant selon la revendication 1, **caractérisée en ce que** sur chaque paroi latérale (2d) du corps d'armoire (2) est prévu seulement un bras de positionnement (5b) avec deux axes de rotation (8b, 8c) pour le mouvement de l'abattant de meuble (3), au moins une courroie (26) supplémentaire, de préférence une courroie dentée (27), étant guidée entre un élément de renvoi (25a), monté immobile en rotation sur le corps d'armoire (2), et l'élément de renvoi (25b), qui est monté rotatif sur l'extrémité libre du bras de positionnement (5b) et qui est destiné à être relié de manière solidaire en rotation avec l'élément de ferrure (6) du côté abattant, le système anti-rotation permettant de bloquer l'élément de renvoi (25b) par rapport au bras de positionnement (5b).

5. Ferrure pour abattant selon la revendication 4, **caractérisée en ce que** le système anti-rotation comporte un verrou (28) agencé sur le bras de positionnement (5b) et pouvant être amené en prise avec l'autre élément de renvoi (25b) de l'élément de ferrure (6) du côté abattant.

6. Ferrure pour abattant selon l'une quelconque des revendications 1 à 5, **caractérisée en ce que** l'évidement (20) s'étend radialement par rapport à l'axe de rotation (8b) du bras de positionnement (5b).

7. Ferrure pour abattant selon l'une quelconque des revendications 1 à 5, **caractérisée en ce que** l'évidement (22) s'étend transversalement par rapport à l'axe de rotation (8b) du bras de positionnement (5b).

8. Ferrure pour abattant selon l'une quelconque des revendications 1 à 7, **caractérisée en ce que** l'élément de verrouillage (15a) est formé par un verrou (15).

9. Ferrure pour abattant selon l'une quelconque des revendications 1 à 7, **caractérisée en ce que** l'élément de verrouillage (21a) est formé par un élément rotatif (21).

10. Ferrure pour abattant selon la revendication 9, **caractérisée en ce que** l'évidement (22) est un creux cylindrique.

11. Ferrure pour abattant selon la revendication 9 ou 10, **caractérisée en ce que** l'élément rotatif (21) comporte un cylindre (21a) avec un évidement (21c) cylindrique qui s'étend transversalement à l'axe (21b) du cylindre.

12. Ferrure pour abattant selon l'une quelconque des revendications 1 à 11, **caractérisée en ce que** l'élément de ferrure (6) du côté abattant comporte un corps de base (6a) pré-monté sur l'abattant (3) et un élément de fixation (6b) relié au bras de positionnement (5a, 5b).

13. Ferrure pour abattant selon la revendication 12, **caractérisée en ce que** l'élément de fixation (6b) peut être relié au corps de base (6a) par l'intermédiaire d'un dispositif de fixation (10a, 10b) amovible, de préférence une liaison mécanique par encochage.

14. Ferrure pour abattant selon la revendication 13, **caractérisée en ce que** la liaison mécanique par encochage est réalisée de telle sorte que l'élément de fixation (6b) peut être accroché dans le corps de base (6a) et être bloqué ensuite avec celui-ci sous l'effet d'un pivotement ouvert.

15. Ferrure pour abattant selon la revendication 13 ou 14, **caractérisée en ce que** l'élément de fixation (6b) est bloqué avec le corps de base (6a), en plus du dispositif de fixation (10a, 10b) amovible, par un système anti-décrochement (15b) séparé.

16. Ferrure pour abattant selon la revendication 15, **caractérisée en ce que** le système anti-décrochement (15b) comporte un verrou (15), qui, de préférence, est sollicité par un ressort et qui, dans une position de service, peut être amené en prise avec un étrier de sécurité (16) disposé ou réalisé sur le corps de base (6a) ou sur l'élément de fixation (6b).

17. Ferrure pour abattant selon la revendication 16, **caractérisée en ce que** le système anti-rotation (15a) est formé par le même verrou (15) que le système anti-décrochement (15b).

18. Ferrure pour abattant selon l'une quelconque des revendications 1 à 17, **caractérisée en ce que** la position relative du bras de positionnement (5a, 5b) par rapport à l'élément de ferrure (6) du côté abattant peut être modifiée par l'intermédiaire d'au moins un dispositif de réglage (11, 12, 13).

19. Ferrure pour abattant selon la revendication 18, **caractérisée en ce que** le bras de positionnement (5a, 5b) peut être réglé en hauteur (H), en inclinaison (α) et/ou en orientation latérale (B) par rapport à l'élément de ferrure (6) du côté abattant par l'intermédiaire dudit au moins un dispositif de réglage (11, 12, 13).

20. Ferrure pour abattant selon la revendication 18 ou 19, **caractérisée en ce que** respectivement un dispositif de réglage (11, 12, 13) séparé est prévu pour le réglage de la hauteur (H), de l'inclinaison (α) et de l'orientation latérale (B).

21. Ferrure pour abattant selon l'une quelconque des revendications 18 à 20, **caractérisée en ce que** ledit dispositif de réglage (11, 12, 13) comporte au moins une vis filetée qui peut être actionnée manuellement ou au moyen d'un élément de réglage, de préférence un tournevis.

22. Ferrure pour abattant selon l'une quelconque des revendications 1 à 21, **caractérisée en ce que** deux bras de positionnement (5a, 5b) sont montés pivotant sur l'élément de ferrure du côté abattant.
